# EUROPÄISCHE PATENTSCHRIFT

(11) **EP 1 726 960 B1**
(45) Veröffentlichungstag und Bekanntmachung des Hinweises auf die Patenterteilung: **11.03.2009**
(21) Anmeldenummer: 05011134.3
(22) Anmeldetag: 23.05.2005
(51) Int. Cl.: G01N 33/569

(54) **Träger und Verfahren zum Nachweis von Anti-Borrelia-Antikörpern sowie Testkit zur Verwendung in der Diagnostik von Lyme-Borreliose-Infektionen**
Carriers and methods for the detection of anti-Borrelia-antibodies and kit for the use in the diagnosis of Lyme-Borrelia infections
Supports et procédés pour la détection des anticorps anti-Borrelia et kit pour utilisation dans le diagnostic de la borreliose de lyme

(43) Veröffentlichungstag der Anmeldung: 29.11.2006
(73) Patentinhaber: Viramed Biotech AG, 82152 Planegg (DE)
(72) Erfinder: Kintrup, Martin, Dr., 82131 Gauting (DE); Helbl, Vera, Dr., 80689 Munich (DE); Furtmayr, Ludwig, Dr., 86989 Steingaden (DE); Kronsteiner, Lilly, Dr., 82152 Planegg (DE)
(74) Vertreter: Grünecker, Kinkeldey, Stockmair & Schwanhäusser Anwaltssozietät

(56) Entgegenhaltungen:
- EP-A- 0 821 237
- DE-A1- 19 847 142
- SCHULTE-SPECHTEL ULRIKE ET AL: "Significant improvement of the recombinant Borrelia-specific immunoglobulin G immunoblot test by addition of VlsE and a DbpA homologue derived from Borrelia garinii for diagnosis of early neuroborreliosis." JOURNAL OF CLINICAL MICROBIOLOGY. MAR 2003, Bd. 41, Nr. 3, März 2003 (2003-03), Seiten 1299-1303, XP002350635 ISSN: 0095-1137

## Beschreibung

Die Erfindung betrifft einen zelllysat-freien Träger und ein Verfahren zum Nachweis von Anti-*Borrelia*-Antikörpern, sowie einen Testkit für die Verwendung in der Lyme-Borreliose-Diagnostik.

Die Lyme-Borreliose ist eine häufige Infektionskrankheit in Europa mit einer Inzidenz, welche jährlich bei schätzungsweise 16 bis 140 pro 100000 Einwohnern liegt. Lyme-Borreliose wird durch die Spirochäte *Borrelia burgdorferi* hervorgerufen, ein schraubenförmiges Bakterium von circa 8 bis 30 µm Länge, welches sowohl Menschen als auch Tiere infizieren kann. Eine Vielzahl von möglichen Zwischenwirten ist beschrieben. In Europa werden die vier Genospezies *B. afzelii, B. garinii, B. b. sensu stricto* und *B. valaisiana* am häufigsten durch Zeckenbisse übertragen.

Der Krankheitsverlauf einer typischen Lyme-Borreliose-Infektion lässt sich im allgemeinen in drei Stadien unterteilen. Das Stadium I tritt mit dem Leitsymptom Erythema (chronicum) migrans unter Bildung einer Hautrötung in Erscheinung, die sich über eine Zeitdauer von Tagen bis Wochen zu einem großen runden Hof um den Zeckenbiss erweitert. Kennzeichnend für das Stadium II ist zumeist eine Neuroborreliose, die vor allem als Meningoradikulitis, Meningitis oder Meningoenzephalitis auftreten kann. Schliesslich treten im Stadium III Lyme-Arthritis, Acrodermatitis chronica atrophicans oder chronisch-progredienter Borrelien-Enzephalomyelitis als häufigsten Manifestationen auf.

Nicht immer werden jedoch alle drei Stadien durchlaufen, sondern es sind durchaus fließende Übergänge und symptomfreie Intervalle möglich. Überdies werden häufig ganze Stadien übersprungen, so dass sich die Erkrankung praktisch in jeder der drei Phasen erstmalig manifestieren kann.

Aufgrund der insgesamt vielfältigen klinischen Symptomatik, welche sehr oft auch anderen Krankheitsformen ähnelt, und dem Umstand, dass die Anamnese des Patienten oftmals keinen Anhaltspunkt auf einen vorangegangenen Zeckenbiss aufweist, ist es geboten, die Diagnose durch Laboruntersuchungsmethoden differenzialdiagnostisch zu ergänzen. Dies ist schon deshalb geboten, weil bei positivem Befund eine längere Antibiotika-Behandlung indiziert ist. Größte Bedeutung in der Labordiagnostik hat der Nachweis von Antikörpern gegen *Borrelia* sp. (IgM, IgG) im Serum, welcher bei neurologischen Manifestationen der Erkrankung zusätzlich auch auf den Liquor des Patienten ausgedehnt werden kann.

Nach dem Prinzip der Stufendiagnostik wird dabei zuerst ein serologischer Erst- oder Suchtest auf IgM- und IgG-Antikörper unter Verwendung von enzymgekoppelten Immunabsorptionstests (ELISA) durchgeführt.

Zur Differenzierung des Antikörpernachweises auf Anti-*Borrelia*-Antikörper wird dem Suchtest ein sogenannter Bestätigungstest angeschlossen. Dieser ist im allgemeinen ein Westemblot, welcher die Antikörperantwort des Patienten auf einzelne Proteine des Bakteriums differenziert anzeigt. Anhand der Bandenmuster auf dem Blot können neben der Aussage, ob Antikörper gegen Borrelia vorliegen oder nicht, gegebenenfalls auch Informationen über die Infektionsphase erhalten werden, da die Immunantwort des Wirtes sich in den drei Stadien der Infektion hinsichtlich Prävalenz und Prädominanz der Antikörper der verschiedenen Ig-Klassen signifikant unterscheidet. In den Frühphasen werden vorwiegend IgM-Antikörper nachgewiesen, während in den Spätphasen überwiegend IgG-Antikörper nachweisbar sind.

Im allgemeinen kann im Bestätigungstest während des Stadiums I der Lyme-Borreliose in circa 20 bis 50 % der Fälle mit einem positiven Antikörpemachweis im serologischen Befund gerechnet werden (WO 91/09870). Entsprechend kann im Stadium II in circa 70 bis 90 % der Fälle mit einem positiven Antikörpernachweis gerechnet werden, und im Stadium III sind Antikörper nahezu immer nachweisbar.

Zum Stand der Technik gehören bereits mehrere Bestätigungsteste, welche entweder von *Borrelia burgdorferi* sensu lato abgeleitete, rekombinante Antigene oder gelelektrophoretisch aufgetrennte Ganzzelllysate im Westernblot-Format verwenden.

So wird in DE 196 29 543 ein diagnostisches Mittel zum Nachweis von Anti-*Borrelia-burgdorferi-Antikörpern* beschrieben, welches auf den von *B. afzelii* abgeleiteten, rekombinanten Antigenen P870, P412, P402, P365, P315, P310, P280, P250, P230 und P130 aufbaut.

In DE 198 47 142 ist beschrieben, wie Lysate von Spirochäten-Stämmen durch Polyacrylamid-SDS-Gelelektrophorese aufgetrennt und nach Übertragung auf Nitrozellulosemembran mit einem Immunserum aus einer Maus kontaktiert werden. Die Antigene, welche unaufgereinigt verwendet werden und nicht bei *In-vitro-*Kultivierung von *Borrelia*-Stämmen exprimiert werden, sondern nur durch *In-vivo*-Kultivierung, haben Molekularmassen im Polyacrylamid-SDS-Gel von ca. 9,5, 18, 19, 30, 32, 33, 62, 70, 80, 90, 100 und 102 kDa.

Die Verwendung von verschiedenen rekombinanten Antigenen für serodiagnostische Immunoblots basierend auf p83/100, p39, OspC und p41 (Flagellin) ist in Wilske et al., Med. Microbiol. Immunol. 1993, 182, 255-270 und Rössler et al., J. Clinic. Microbiol. 1997, 35, 2752-2758 beschrieben.

In der Offenlegungsschrift DE 40 18 988 ist ein Westernblot-Assay mit verschiedenen von anderen aus Borrelia burgdorferi stammenden Proteinen beschrieben. Die verwendeten Antigene sind rekombinant und haben Molekularmassen von 17, 22, 41, und 100 KDa.

Ein Immunoblot für rekombinantes Osp17 und p58 ist in B. Wilske et al. Med. Microbiol. Immunol. 1999, 188, 139-144 beschrieben.

Ein weiterer rekombinanter Immunoblot, welcher zusätzlich VlsE, DbpA und ein OspC-Homolog enthält, ist von der gleichen Arbeitsgruppe in U. Schulte-Spechtel et al., J. Clinic. Microbiol. 2003, 41, 1299-1303 beschrieben.

Ähnlich ist in WO 91/09870 beschrieben, wie drei rekombinante Antigene, p41 (rekomb.), OspA (rekomb.) und pC (rekomb.) im ELISA-Verfahren zur Detektion von IgM-Antikörpern und IgG-Antikörpem gegen Borrelia in Patienten mit Erythema migrans eingesetzt werden können.

Nachteilig bei den Bestätigungstesten des Stands der Technik ist, dass sie eine Borreliose-Infektion im Stadium I der Erkrankung in nur 20 bis 50 % der Fälle nachweisen (siehe beispielsweise WO 91/09870). Da eine vollständige klinische Heilung nach dem Erreichen eines späteren Erkrankungsstadiums häufig nicht möglich ist, sollte jedoch eine beginnende Lyme-Borreliose-Infektion möglichst frühzeitig diagnostiziert werden.

Die Zuordnung der *Borrelia*-Antigene auf Westernblotstreifen unter Verwendung des Vollzelllysats ist ferner abhängig vom verwendeten Gelsystem und kann oftmals nur von Fachpersonal ausgewertet werden.

Diese Teste besitzen durchweg heterogene diagnostische Wertigkeiten, die dem Anwender, das heißt dem medizinischen Untersuchungslabor, und dem anfordernden Arzt häufig nicht bekannt sind.

Die Bestätigungsteste des Standes der Technik, welche gelelektrophoretisch aufgetrennte Ganzzelllysate im Westernblot-Format verwenden, sind teilweise sehr schwierig zu beurteilen, da sich sowohl spezifische als auch unspezifische Banden (Proteine) auf dem Nitrozellulosestreifen befinden, und sich zudem die Banden (Proteine) aufgrund des elektrophoretischen Herstellungsprozesses nicht immer an der gleichen Position befinden, sondern in ihrer Lage auf dem Träger variieren.

Die Beurteilung von spezifischer oder unspezifischer Reaktion und die genaue Zuordnung der Banden erfordert hier sehr erfahrenes Fachpersonal und kostet viel Zeit.

Auf der anderen Seite stehen für Bestätigungsteste des Standes der Technik mit einzeln aufgetragenen *Borrelia*-Proteinen bisher nur rekombinante Proteine zur Verfügung, die aufgrund der geringeren Antigenität deutlich geringere Sensitivität als der klassische Westernblot besitzen. Die Diagnose mit diesen rekombinanten Testen liefert daher häufig qualitativ unzureichende Ergebnisse.

Sowohl die Teste, welche rekombinanten Antigenen verwenden, als auch die Teste mit Ganzzelllysate im Westemblot-Format sind oft umständlich hinsichtlich der Verfahrensführung/Handhabung und für den Routinebetrieb und insbesondere zur Verwendung im Hochdurchsatzverfahren (HTS-Verfahren) meist nicht geeignet.

In EP0821237 werden zum einen mit rekombinanten Antigenen bestückte Träger und zum anderen Zelllysat-enthaltende, via Western-Blot erstellte, mit Wildtyp-Antigenen bestückte Träger zum *Borellia*-Nachweis offenbart.

Aufgabe der vorliegenden Erfindung ist es, einen (diagnostischen) Träger zur Verfügung zu stellen, welcher zum Nachweis von Anti-Borrelia-Antikörpem mit hoher Sensitivität und Spezifität dient und welcher die umfassende Spezifität und Sensitivität des klassischen Zelllyst-Westemblotformates mit der Benutzerfreundlichkeit und Beurteilungssicherheit der Teste mit diskret aufgetragenen einzelnen *Borrelia*-Proteinen - bisher nur als rekombinante Proteine verfügbar - kombiniert und damit die oben erwähnten Unzulänglichkeiten bestehender Teste nicht mehr aufweist.

Gleichfalls ist eine Aufgabe der vorliegenden Erfindung, ein Verfahren zum Nachweis von Anti-*Borrelia*-Antikörpern bereitzustellen, welcher einfach zu standardisieren ist unter Verwendung der Wildtyp-*Borrelia*-Antigene. Die mit dem beschriebenen Verfahren aufgereinigten Antigene haben den Vorteil, dass ihre posttranslationalen Modifikationen denen der natürlichen Krankheitserregern entsprechen. Damit ergibt sich ein Vorteil gegenüber den rekombinanten Antigenen: da rekombinante Antigene aus heterologen Zellen isoliert werden, weisen sie keine *Borrelia*-spezifischen posttranslationalen Modifikationen auf. Um die Toxizität von hoch exprimierten Membranproteinen zu reduzieren, stellen rekombinante Proteine häufig verkürzte Derivate dar, denen häufig Epitope gegenüber den Wildtyp-Proteinen aus den Vollzelllysaten fehlen.

Eine positionsspezifische Auftragung der aufgereinigten Antigene auf einem Träger führt zu einer einfacheren Auswertung und sicheren Identifizierung der Antigen-Antikörperkomplexe. Die Auswertung kann im Unterschied zu Vollzelllysaten auch von Nichtexperten sicher und zuverlässig vorgenommen werden. Die klare Ablesbarkeit des Testes stellt einen klaren Vorteil gegenüber dem Westemblot dar.

Aufgabe ist weiterhin die Bereitstellung von *borrelia-burgdorferi*-spezifischen Antigenen zur Entwicklung eines diagnostischen Trägers zum Nachweis von Anti-Borrelia-Antikörpern, wobei die Antigene vorzugsweise: a) aus homologen *Borrelia*-Stämmen isoliert oder extrahiert werden, b) eine hohe Antigenität mit detektierbaren Antikörpern im frühen Stadium der Immunantwort besitzen und c) möglichst geringe Kreuzreaktivität mit Antigenen aufweisen, welche nicht spezifisch für *Borrelia burgdorferi* sensu lato sind.

Bei der Entwicklung eines Diagnostik-Testes ist insbesondere die Auswahl der Antigene, die eine hohe Sensitivität und Spezifität für den Nachweis im frühen Infektionsstadium aufweisen, von entscheidender Bedeutung.

Gegenstand der Erfindung ist ein zelllysat-freier Träger zum Nachweis von Anti-*Borrelia*-Antikörpern, umfassend mindestens zehn aus der Membranfraktion von *Borrelia burgdorferi* sensu lato abgeleitete und mittels präparativer SDS-Polyacrylamid-Gelektrophorese aufgereinigte Wildtyp-Antigene, welche an Anti-*Borrelia*-Antikörper binden können und auf unterschiedlichen, voneinander getrennten Positionen des Trägers vorliegen, wobei die aufgereinigten Wildtyp-Antigene in definierter Ortsauflösung oder Positionierung aufgebracht sind und wobei die aufgereinigten Wildtyp-Antigene unabhängig voneinander auf den Träger beladen werden; wobei Wildtyp-Antigene im Unterschied zu rekombinanten Antigenen posttranslationale Modifikationen aus Borrelia-Zellen aufweisen.

Unter von *Borrelia burgdorferi* sensu lato abgeleiteten Antigenen *(Borrelia-*Antigene) werden erfindungsgemäß Präparationen verstanden, die diagnostisch relevante Antigene von *Borrelia burgdorferi* sensu lato enthalten. Dabei handelt es sich vorzugsweise um Antigene mit hoher Spezifität für *Borrelia burgdorferi* sensu lato sowie hoher Reaktivität in den verschiedenen Stadien der Lyme-Borreliose.

Besonders vorzugsweise werden *Borrelia*-Antigene von solchen *Borrelia-*Stämmen verwendet, die mit den im Anwendungsgebiet des Testes vorkommenden natürlichen *Borrelia*-Stämmen eine so hohe Antigen-Homologie aufweisen, dass Borreliose-Infektionen mit hoher Sensitivität erfasst werden. Für Deutschland ist vorzugsweise die Verwendung der Stämme B. *afzelii,* B. *garinii,* und *B. b.* sensu *stricto* als Antigen-Quelle geeignet.

Erfindungsgemäß ist ein Wildtyp-Antigen ein unter homologer Kontrolle exprimiertes Antigen, welches dem in der Zelle vorkommenden natürlichen Antigen entspricht. Unter Wildtyp-Protein werden insbesondere nicht rekombinante Antigene verstanden, welche mit Hilfe von einem gentechnologischen Verfahren gewonnen wurden, das heißt beispielsweise unter Einschleusung von heterologer DNA in die Produzentenzelle. Im Unterschied zu rekombinanten Antigenen weisen Wildtyp-Antigene posttranslationale Modifikationen auf. Sie liegen entweder in einer gefalteten oder in einer komplett oder teilweise denaturierten Modifikation vor.

Eine Aufreinigung von Borrelia-Antigenen mit posttranslationalen Modifizierungen aus Borrelia-Zellen führte erfindungsgemäß zu einer besseren Reaktivität als entsprechende rekombinante Antigene und zu einer vergleichbaren Reaktivität der elektrophoretisch aufgetrennten Antigene im Westernblot.

Vorzugsweise sind die *Borrelia*-Antigene partiell denaturiert und gewährleisten die optimale Präsentation von linearen Epitopen.

Erfindungsgemäß bedeutet aufgereinigt, dass das Wildtyp-Antigen in einer Reinheit von mindestens 50 Gew.-%, und vorzugsweise mindestens 90 Gew.-%, und besonders vorzugsweise mindestens 95 Gew.-% vorliegt.

In einer vor allen bevorzugten Ausführungsform ist die Reinheit mindestens 99 Gew.-%, wobei die Restbestandteile ausschließlich aus Wasser und/oder anorganischen Salzen bestehen.

Ein "zelllysat-freier Träger" ist ein Träger, der kein für Ganzzellysate typisches Muster an Komponenten aufweist. Solche Ganzzelllysate enthalten eine Reihe von *Borrelia* unspezifischen und nicht-relevanten Komponenten, auch Proteinen, wie z.B. zelluläre Proteine. Diese unspezifischen und nicht-relevanten Komponenten umfassen keine *Borrelia burgdorferi* sensu lato abgeleiteten Antigene und sind im "zelllysat-freien Träger" der vorliegenden Erfindung nicht vollständig enthalten. Folglich enthalten die "zelllysat-freien Träger" der vorliegenden Erfindung zumindest eine solche unspezifische oder nicht-relevanten Komponente nicht, die in Ganzzelllysaten typischerweise enthalten ist. Bevorzugterweise ist der "zelllysat-freie" Träger lipid- und kohlenhydrat-frei. Mehr bevorzugt enthält der "zelllysat-freie Träger" der vorliegenden Erfindung keine der besagten unspezifischen und nicht-relevanten Komponenten.
In einer bevorzugten Ausführungsform ist der Träger so gefertigt, dass die mindestens zwei von *Borrelia burgdorferi* abgeleiteten Wildtyp-Antigene aus der Gruppe bestehend aus 14 kD-, 17 kD-, 21 kD-, 25 kD-, 30 kD-, 39 kD-, 41 kD-, 43 kD-, 58 kD-, 83 kD-Protein und DbpA ausgewählt sind.

In einer bevorzugten alternativen Ausführungsform sind die mindestens zwei von *Borrelia burgdorferi* abgeleiteten Wildtyp-Antigen aus der Gruppe bestehend aus 18 kD-, 25 kD- (OspC), 28 kD-, 30 kD-, 31 kD-, 39 kD-, 41 kD-, 45 kD-, 58 KD-, 66 kD-, 83 (93) kD-Protein und DbpA ausgewählt.

In einer besonders bevorzugten Ausführungsform umfasst der erfindungsgemäße Träger mindestens 2, oder mindestens 4, oder mindestens 6, oder mindestens 8 oder alle unterschiedlichen Antigene ausgewählt aus der Gruppe bestehend aus 14 kD-, 17 kD-, 21 kD-, 25 kD-, 30 kD-, 39 kD-, 41 kD-, 43 kD-, 58 kD-, 83 kD-Protein und DbpA oder ausgewählt aus der Gruppe bestehend aus 18 kD-, 25 kD- (OspC), 28 kD-, 30 kD-, 31 kD-, 39 kD-, 41 kD-, 45 kD-, 58 KD-, 66 kD-, 83 (93) kD-Protein und DbpA.

In einer weiteren Ausführungsform umfasst der Träger zusätzlich mindestens ein weiteres Antigen, welches rekombinant ist und von *Borrelia burgdorferi* sensu lato abgeleitet ist.

Das mindestens eine weitere Antigen kann erfindungsgemäß ein vollständiges oder trunkiertes rekombinantes Protein von *Borrelia burgdorferi* sensu lato sein. Vorzugsweise sollen die rekombinanten Antigene gut gereinigt vorliegen.
In einer bevorzugten Ausführungsform wird das mindestens eine von *Borrelia burgdorferi* sensu lato abgeleitete rekombinante Antigen aus der Gruppe bestehend aus 14 kD-, 17 kD-, 18 kD-, 21 kD-, 25 kD- (OspC), 28 kD- 30 kD-, 31 kD-, 39 kD-, 41 kD-, 43 kD-, 45 kD-, 58 kD-, 66 kD- und 83 kD (93)-Protein, VlsE und DbpA ausgewählt.

In einer besonders bevorzugten Ausführungsform des Testträgers wird rekombinantes VlsE als zusätzliches Testreagenz verwendet.

In einer bevorzugten Variante des Trägers ist das mindestens eine weitere von *Borrelia burgdorferi* abgeleitete rekombinante Antigen ein aufgereinigtes Antigen. Erfindungsgemäß bedeutet aufgereinigt, dass das Antigen in einer Reinheit von mindestens 50 Gew.-% vorliegt, vorzugsweise in einer Reinheit von mindestens 90 Gew.-%, oder insbesondere mindestens 95 Gew.-%.

In einer besonderes bevorzugten Ausführungsform liegt das mindestens eine weitere von *Borrelia burgdorferi* abgeleitete rekombinante Antigen in einer Reinheit von mindestens 99 Gew.-% vor. Es ist erfindungsgemäß bevorzugt, dass die Restbestandteile des Antigens ausschließlich aus Wasser und/oder anorganischen Salzen bestehen.

In einer alternativen Ausführungsform umfasst der Träger mindestens 12 unterschiedliche von *Borrelia burgdorferi* sensu lato abgeleitete aufgereinigte Wildtyp-Antigene.

Erfindungsgemäß sollen mindestens 2, oder mindestens 3, oder mindestens 4, oder mindestens 6, oder mindestens 8, oder mindestens 10, oder mindestens 12, oder alle der von *Borrelia burgdorferi* sensu lato abgeleitete aufgereinigte Wildtyp-Antigene in unterschiedlichen Konzentrationen auf dem Träger vorliegen.

Jedes einzelne Wildtyp-Antigen kann an die Richtlinien der nationalen Referenzzentren unabhängig angepasst bzw. titriert werden. Bei einer solchen Ausführungsform des erfindungsgemäßen Trägers ist von Vorteil, dass ein Über- oder Unterladen mit den Wildtyp-Proteinen ausgeschlossen werden kann. Dies ist bei Verwendung von Westernblot-Systemen mit elektrophoretisierten Ganzzell-Lysaten häufig der Fall, da keine unabhängige Titration oder Beladung der Wildtyp-Proteine vorgenommen werden kann. Ein rekombinanter Westemblot ist zwar prinzipiell titrierbar, jedoch findet keine definierte Positionierung oder Ortsauflösung statt.

Durch die definierte Auftragung aufgereinigter Wildtyp-Antigene und dem Wegfall der Elektrophorese kann einwandfrei entschieden werden, welches Antigen auf dem Träger reagiert.

Erfindungsgemäß umfasst der Träger ein Antigen in einer Konzentration, wenn das Antigen auf dem Träger mit mindestens einem Antigen-Molekül vorliegt. Es ist jedoch bevorzugt, dass auf dem Träger mindestens ein Antigen in einer Konzentration und Menge vorliegt, welche durch Analyseverfahren, die der Fachmann kennt, nachweisbar ist und die für die Antikörperdiagnostik geeignet ist.

Erfindungsgemäß umfasst der Träger zusätzlich mindestens eine Kontrollsubstanz. Diese ist dazu geeignet, die sachgemäße Durchführung des erfindungsgemäßen Verfahrens anzuzeigen oder Daten zu liefern, die zur Auswertung von Testergebnissen hilfreich sind.

In einer bevorzugten Ausführungsform ist die mindestens eine Kontrollsubstanz eine *Borrelia*-unabhängige Konjugatkontrollsubstanz, vorzugsweise ausgewählt aus der Gruppe bestehend aus IgA-, IgG- und IgM-Konjugatkontrollsubstanz. Erfindungsgemäß kann die Kontrollsubstanz eine Cutoff-Kontrolle sein, die auf dem Träger aufgebracht ist oder als biologisches Material unabhängig eingesetzt ist.

Eine Bande mit schwächerem Signal als eine solche Cutoff-Kontrolle wird nicht gewertet, während ein gleichstarkes oder stärkeres Signal zu einer gültigen Bande führt. Die Cutoff-Kontrolle kann beispielsweise eine Verdünnung von gereinigtem humanen Immunoglobulin oder alkalische Phosphatase sein. Die Cutoff-Kontrollbande ist als Grenzwertanzeige gestaltet, die angibt, ab welcher Signalintensität das Testergebnis positiv oder negativ oder gegebenenfalls auch fraglich zu bewerten ist.

In einer bevorzugten Ausführungsform umfasst der diagnostische Träger folgende Serumkontrollen: a) eine oder mehrere Negativkontrollseren, b) mindestens ein Positivkontrollserum zur Erkennung wenigstens einer, vorzugsweise aller diagnostisch relevanten Banden, c) mindestens ein Cutoff-Kontrollserum zur Erkennung des grenzwertigen Bereiches der Bandenintensität, wobei eine Bande erfindungsgemäß dann als reaktiv bewertet wird, wenn ihre Intensität mindestens derjenigen der Cutoff-Kontrolle entspricht.

Unter *Borrelia*-Banden werden erfindungsgemäß diagnostisch relevante, reaktive und/oder erkennbare Banden nach Testdurchführung mit einer reaktiven Probe verstanden. Erfindungsgemäß werden unter *Borrelia*-Banden ebenfalls reaktive Dots oder Lines verstanden, falls der Träger im Dot-Blot- oder Line-Blot-Format ausgestaltet ist.

In einer bevorzugen Ausführungsform umfasst der Träger eine Kontaktkontrolle, die eine Aussage darüber zulässt, ob der Träger mit einer zu testenden biologischen Probe in Kontakt getreten ist. Da die zu testende biologische Probe vorzugsweise eine wässrige Probe ist, d. h. Wasser als Hauptbestandteil (d.h. 10 bis 99 Gew.-%) oder Nebenbestandteil (d.h. 1 bis 10 Gew.-%) enthält, ist in einer Ausführungsform die Kontaktkontrolle als Feuchtigkeitskontrolle ausgestaltet. Entsprechende Substanzen, die als Kontaktkontrolle verwendet werden können, sind dem Fachmann bekannt. Beispielsweise können dehydriertes Kupfer(II)sulfat oder Kobaltchlorid als einfache Substanzen zum Nachweis der Benetzung mit einer wässrigen biologischen Probe benutzt werden.

In einer besonders bevorzugten Ausführungsform ist die Kontaktkontrolle als eine Serumkontrolle ausgestaltet. Dadurch kann, falls die biologische Probe Serum ist, der Kontakt des Trägers mit der biologischen Probe nachgewiesen werden. Als Serumkontrolle kommt insbesondere Protein A oder eine entsprechende andere serumbindende Substanz in Betracht.

In einer vorteilhaften Ausführungsform ist der Träger so gefertigt, dass er als ein fester Träger hergestellt ist, an dem die Antigene immobilisiert sind. Der feste Träger sollte vorzugsweise aus Platte, Streifen, Membran, Filter, Papier, Film oder Perle ausgewählt sein.

Bei einer besonders bevorzugten Ausführungsform ist der Träger als Teststreifen ausgestaltet, insbesondere in einem Format, das den Einsatz in herkömmlichen Verfahren der Immundiagnostik erlaubt. Werden im diagnostischen Test gereinigte Wildtyp-Antigene und/oder gereinigte rekombinante oder gentechnisch hergestellte Antigene als Reagenskomponenten eingesetzt, so werden die isoliert vorliegenden Antigene direkt an den festen Träger immobilisiert, vorzugsweise direkt an den Platten, Streifen, Membranen, Filter, Papier, Film oder Perlen. Als Platten kommen insbesondere Mikrotiterplatten in Frage. Die Perlen können auch Kügelchen aus verschiedenem Material sein, an welchem die Antigene aufgetragen oder immobilisiert sind.

Bei einer weiteren bevorzugten Ausführungsform ist der Träger als Westernblot ausgestaltet. Dabei umfasst der Träger die Reagenzkomponenten in immobilisierter Form.
Prinzipiell sind alle Materialien zum Aufbau des Trägers geeignet, die dem Fachmann zur Immobilisierung von Antigenen bekannt sind.

In einer besonders bevorzugten Ausführungsform besteht der Träger jedoch aus Material ausgewählt aus der Gruppe von Nitrocellulose, Polyvinylidendifluorid (PVDF), Polyamid, Celluloseacetat und Polystyren. Für den Fall, dass Polyamid als Material verwendet wird, ist Nylon die bevorzugte Wahl. Nitrocellulose ist jedoch aufgrund ihrer physikalischen Oberflächeneigenschaften das zur Immobilisierung der Antigene vor allen zu bevorzugende Material. Insbesondere hat sich gezeigt, dass beim Einsatz von Nitrocellulose ein besonders gutes Signal-Rauschverhältnis für alle diagnostisch relevanten Bandensignale erreicht werden kann.

Der Träger kann weiterhin ein beschichteter Träger sein. Dann besteht der beschichtete Teil des Trägers vorzugsweise aus einem festen Material mit solchen Eigenschaften, die für eine Beschichtung geeignet ist. Diese sind dem Fachmann bekannt. Die Beschichtung des Trägers umfasst dabei vorzugsweise ein Material ausgewählt aus der obigen Gruppe von erwähnten Materialien. Vorzugsweise sind der beschichtete Teil und die Beschichtung so miteinander verbunden, dass ein unbeabsichtigtes Lösen beider Teile nicht möglich ist.

In einer alternativen Ausführungsform kann jedoch auch ein beschichteter Träger verwendet werden, deren Beschichtung gerade ablösbar mit dem beschichteten Teil verbunden ist, wenn die Beschichtung beispielsweise in einem standardisierten Verfahren weiter zur Analyse dienen soll.

Die verwendeten Antigene können erfindungsgemäß unmittelbar auf den beschichteten Träger aufgetragen werden oder zusammen mit dem Beschichtungsmaterial auf den zu beschichtenden Teil aufgetragen werden.

In einer bevorzugten Ausführungsform ist der Träger mit Hilfe einer Stripetechnik hergestellt, welche die aufgereinigten Antigene auf bestimmte Positionen aufträgt und damit die genaue Zuordnung der Banden gewährleistet. Durch die positionsspezifische Auftragung der aufgereinigten Antigene auf dem Träger wird eine einfache Auswertung und sichere Identifikation der Antigen-Antikörper-Komplexe ermöglicht. Dadurch kann die Auswertung im Unterschied zu Vollzelllysaten auch von Nichtexperten sicher und zuverlässig vorgenommen werden.

Die gute Ablesbarkeit des Testes stellt einen klaren Vorteil gegenüber dem Westernblot dar, da eine Verwendung im Rahmen von HTS-Screening-Verfahren mit automatisierter Betriebsführung möglich ist.

Erfindungsgemäß kann der Träger wie oben beschrieben in der Diagnostik von Lyme-Borreliose-Infektionen oder zur Verlaufskontrolle einer Lyme-Borreliose-Infektion verwendet werden.

Gegenstand der vorliegenden Erfindung ist ein Verfahren zum Nachweis von Anti-*Borrelia*-Antikörpern. Dieses Verfahren umfasst erfindungsgemäß folgende Schritte: 1) Bereitstellen eines Trägers wie oben beschrieben, 2) Inkubieren einer biologischen Probe, die möglicherweise Anti-Borrelia-Antikörpem enthält, mit dem Träger unter Bedingungen, die eine Ausbildung mindestens eines Antigen-Antikörper-Komplexes erlauben; und 3) Bestimmen der Anwesenheit von Anti-*Borrelia*-Antikörpern in der Probe durch Nachweis mindestens eines Antigen-Antikörper-Komplexes.

Das Verfahren ist dabei ebenfalls geeignet, ein Mittel zur Diagnostik und/oder Verlaufskontrolle einer Borreliose-Infektion darzustellen. Dazu ist die biologische Probe eine Patientenprobe, das heißt eine Probe die einem menschlichen oder tierischen Körper entnommen wurde oder aber eine aus einem menschlichen oder tierischen Körper entnommene, weiterverarbeitete Probe.

Erfindungsgemäß können mit dem Verfahren oder dem Träger Antikörper gegen verschiedene Genospecies von *Borrelia burgdorferi* sensu lato erkannt werden. Die erkannten Antikörper umfassen, sind jedoch nicht limitiert auf Antikörper gegen folgende Species: *B. andersonii, B. afzelii, B. b. sensu stricto, B. garinii, Borrelia* Group DN127, *Borrelia* Group DNPotiB2, *Borrelia* Group VS116, *B. japonica, B. valaisiana.* Vorzugsweise dient das Verfahren oder der Träger zur Detektion von Anti-*Borrelia*-Antikörpern gegen Stämme, die erwiesenermaßen humanpathogen sind, insbesondere Stämme der Species *B. afzelii, B. garinii, B. b. sensu stricto,* oder Stämme für die eine Humanpathogenität diskutiert wird, wie beispielsweise *B. valaisiana.*

Sind Kontrollsubstanzen auf dem Träger enthalten, so kann mit diesen die sachgemäße Durchführung des erfindungsgemäßen Verfahrens angezeigt werden. Beispielsweise zeigt eine Kontaktkontrolle an, ob der Träger lange genug mit der biologischen Probe in Kontakt getreten ist. Eine Serumkontrolle zeigt entsprechend an, ob während des Verfahrens der Träger mit einem Serum in Kontakt getreten ist. Erfindungsgemäß lassen die Kontrollsubstanzen auch Aussagen darüber zu, ob die Inkubation der biologischen Probe mit dem Träger für eine Ausbildung mindestens einen Antikörper-Antigen-Komplexes hinreichend lange war. In diesem Falle handelt es sich bei der Kontrollsubstanz um eine Inkubationszeitkontrolle bzw. Cutoff-Kontrolle.

Vorzugsweise ist das Nachweisverfahren für Anti-*Borrelia*-Antikörper so gestaltet, dass es alle Vorgaben hinsichtlich interner und externer Qualitätssicherung nach den Deutschen Industrienormen DIN 58967-40 und DIN 58967-10 erfüllt. Besonders bevorzugt ist, dass das Nachweisverfahren zusätzlich die Norm MIQ 2000 und die US-amerikanische Norm CDC erfüllt.

In einer bevorzugten Ausführungsform ist das Verfahren so gestaltet, dass mindestens ein Antigen spezifisch mit einem Anti-*Borellia*-Antikörper reagiert.

Die biologische Probe zur Verwendung in dem Verfahren kann von einem Tier oder Menschen abgeleitet sein. Besonders bevorzugt ist, dass es sich um eine Probe von einem Menschen handelt.

Vorzugsweise handelt es sich bei der biologischen Probe um eine flüssige Probe. In diesem Fall kommen insbesondere Blut-, Plasma-, Serum-, Urin-, Speichel-, Liquor- oder Synovialflüssigkeit in Frage. Besonders vorzugsweise ist die biologische Probe humanes Blut, Serum oder Liquor cerebrospinalis. Die biologische Probe kann auch eine weiterverarbeitete biologische Probe sein. Vorzugsweise ist dann die Weiterverarbeitung so gestaltet, dass die diagnostisch relevanten Anti-Borrelia-Antikörper im Vergleich zur ursprünglichen Probe aufkonzentriert werden, oder in höherer Reinheit vorliegen. Verfahren zur Weiterverarbeitung der biologischen Probe sind dem auf diesem Gebiet tätigen Fachmann bekannt.

Erfindungsgemäß kann die biologische Probe ebenfalls eine verflüssigte Probe sein, welche aus einer Gewebeprobe oder einem Bioptat, vorzugsweise Hautbioptat, Gelenkbioptat, Synovialbioptat durch ein Weiterverarbeitungsverfahren gewonnen wurde.

Zur Detektion eines Antigen-Antikörper-Komplexes wird in der Diagnostik von Lyme-Borreliose-Infektionen der erfindungsgemäße Testkit verwendet, welches auch zur Verlaufskontrolle einer Lyme-Borreliose-Infektion dienen kann. Im Gegensatz zum Nachweisverfahren mittels PCR kann mit diesem ein eindeutiger Nachweis eines Antigen-Antikörper-Komplexes erfolgen. Ein solcher Testkit enthält neben dem Träger auch mindestens eine Substanz zum Nachweis eines Antigen-Antikörper-Komplexes und/oder mindestens eine Vorrichtung zum Nachweis eines Antigen-Antikörper-Komplexes.

Im Rahmen der Stufendiagnostik zur Diagnose von Lyme-Borreliose ist es möglich, nach einem vorgeschalteten Suchtest (z. B. ELISA, Immunfluoreszenztest) mit dem erfindungsgemäßen Träger, Verfahren und Testkit sowohl die serologische Bestätigungsreaktion, als auch die serologische Verlaufskontrolle durchführen zu können. Die Testdaten liefern einen Teilaspekt für die Entscheidung der Behandlungsbedürftigkeit durch den behandelnden Arzt.

In einer bevorzugten Ausführungsform enthält der Testkit weiterhin eine Anweisung zur Auswertung der Testergebnisse, welche mit dem Testkit gewonnen werden. Diese Anweisung soll es dem Anwender ermöglichen, Aussagen über die Anwesenheit von Anti-*Borrelia*-Antikörpern in der biologischen Probe zu treffen. Die Anweisung ist vorzugsweise so gestaltet, dass Regeln oder Kriterien vorgegeben sind, die eine Aussage darüber ermöglichen, ob die Anwesenheit von Anti-*Borrelia*-Antikörpern 1) positiv ist, falls Anti-*Borrelia*-Antikörpern anwesend sind, oder 2) negativ ist, falls Anti-Borrelia-Antikörpern im wesentlichen nicht anwesend sind, oder 3) das Ergebnis fraglich ist, falls keine Aussage über die Anwesenheit von Anti-Borrelia-Antikörpern getroffen werden kann.

In einer anderen Ausführungsform enthält die Anweisung entsprechend Regeln und Kriterien darüber, wie Aussagen über Konzentrationen von Anti-Borrelia-Antikörpern in der biologischen Probe gewonnen werden können.

In wieder einer anderen Ausführungsform ermöglicht der Träger zusammen mit der Anweisung zusätzliche Aussagen zu treffen, welche Art von Antikörpern, vorzugsweise klassifiziert nach IgG-, IgM- oder lgA-Antikörpern in der biologischen Probe vorliegen. Besonders bevorzugt lässt die Anweisung auch Aussagen darüber zu, in welchen Konzentrationen die verschiedenen Antikörper vorliegen.

In einer weiteren Ausführungsform enthält die Anweisung Informationen darüber, wie der Testkit zur Ermittlung von Aussagen verwendet werden kann, ob eine Testperson, von der die zu untersuchende biologische Probe entnommen wurde, mit *Borrelia burgdorferi*-Erregern infiziert ist.

In noch einer alternativen Ausführungsform enthält die Anweisung Informationen, wie der Testkit zur Verlaufskontrolle einer Lyme-Borreliose-Infektion eingesetzt werden kann. Insbesondere enthält die Anweisung dann Regeln oder Kriterien darüber, wie die Daten zu dem Krankheitsbild korreliert werden können. Vorzugsweise wird dabei eine Grobeinteilung in die drei Phasen der Lyme-Borreliose-Infektion möglich sein. Die Aussage basiert dann erfindungsgemäß darauf, über das Vorhandensein von jeweils IgG-, IgM- und/oder IgA-Antikörpem und falls vorhanden, vorzugsweise auch die Konzentrationen der jeweiligen Antikörper, Rückschlüsse auf das Stadium zu treffen, in welchem sich eine Testperson mit Lyme-Borreliose-Infektion befindet.

Ebenfalls kann die Anweisung Informationen darüber enthalten, wie der Testkit über einen längeren Zeitraum zur Verfolgung einer Lyme-Borreliose-Infektion angewendet werden kann.

Die nachfolgenden Ausführungsbeispiele sollen die vorliegende Erfindung näher erläutern, ohne jedoch limitierend zu wirken. Die Gegenstände der Erfindung sind in der Ansprüchen definiert.

### Beispiel 1:

### Präparation von Membranfraktionen

Alle Schritte wurden bei 4 °C durchgeführt. *Borrelia burgdorferi sensu lato* Zellen wurden durch 20 min Zentrifugation bei 7000 g sedimentiert. Das Pellet wurde zweimal mit gekühltem PBS-Puffer gewaschen und das Feuchtgewicht bestimmt. Die gewaschenen Zellen wurden in 10 ml gekühltem PBS-Puffer pro g Feuchtgewicht suspendiert und durch 2 x 10 min Sonifikation aufgeschlossen (Branson Sonifier). Durch 60 min Zentrifugation bei 140 000 g wurden lösliche Proteine im Überstand erhalten und verworfen. Das Pellet wurde zweimal wie oben beschrieben in PBS suspendiert, sonifiziert und zentrifugiert. Das gewaschene Pellet enthält die Membranfraktion sowie weitere unlösliche Zellbestandteile.

### Beispiel 2:

### Isolierung von Membranproteinen durch präparative Gelelektrophorese

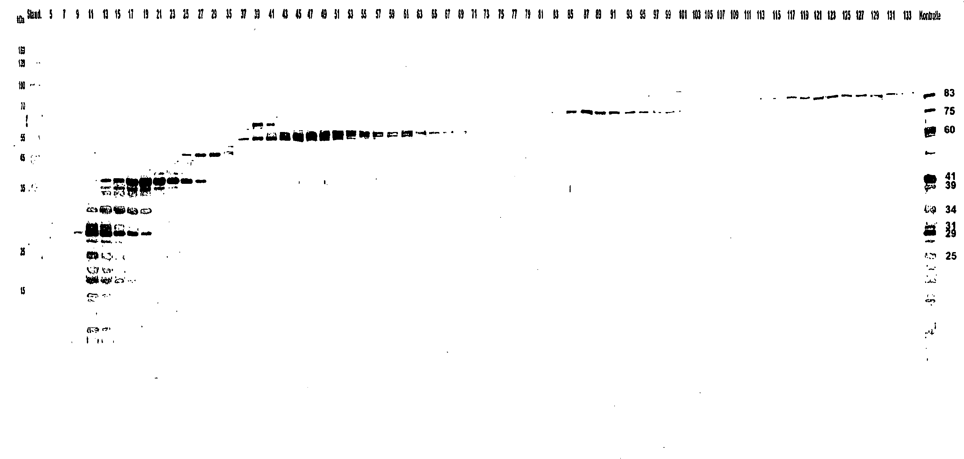

Die Membranfraktion wurde in SDS-Polyacrylamidgelelektrophorese (SDS-PAGE) Probenpuffer (60 mM TrisCl, pH 6.8, 2% SDS, 10% Glycerin, 0,025% Bromphenolblau und 5% Mercaptoethanol) suspendiert und durch Inkubation bei 100 °C gelöst, so dass die Proteinkonzentration etwa 10 mg/ml betrug. Die solubilisierten Proteine wurden durch kontinuierliche Elutions-SDS-Gelelektrophorese in einer "PrepCell" (Model 491, Bio-Rad) in einem Gelsystem nach Laemmli (Laemmli, U K (1970), Nature 227: 680-5) fraktioniert. Dabei wurde, abhängig von der apparenten molekularen Masse des zu isolierenden Antigens, ein 6 cm hohes 7 bis 15%iges Trenngel und ein 2 cm hohes 5%iges Sammelgel in einer Gelröhre mit 37 mm Innendurchmesser verwendet. Die Proteine wurden 14 bis 20 h bei einer konstanten Leistung von 12 W getrennt und mit Elutionspuffer (25 mM TrisCl, pH 8,3, 0.1% SDS) bei einer Flussrate von 90 ml/h eluiert. Unmittelbar nach Elution der Bromphenolblau-Front wurde das Eluat in 3 ml Fraktionen gesammelt. Die UV-Absorption des Eluates lieferte keine aufgelösten Gipfel, die zur Identifizierung der einzelnen Proteine dienen könnten. Daher wurden die einzelnen Fraktionen durch analytische SDS-PAGE und Westemblot untersucht. Die Proteinmenge reicht nicht in allen Fällen für einen Nachweis auf der Membran durch Ponceau S oder im Gel durch Coomassie Brillant Blue aus. Durch geeignete mono- oder oligospezifische Seren konnten die Zielantigene jedoch immunologisch in den entsprechenden Fraktionen nachgewiesen werden (Abb. 1).

### Beispiel 3:

### Umpuffern und Aufkonzentrieren der extrahierten Borrelia-Antigene

Die nach Analyse der PrepCell-Läufe als geeignet ausgesuchten Fraktionen wurden vereinigt und umgepuffert sowie aufkonzentriert. Hierfür wurden Konzentratoren der Firma Vivascience AG (Hamburg, Deutschland) nach den Angaben des Herstellers verwendet (Vivaspin 15, Nr. VS15RH1, 5 kDa-Membran). Die Lösung aus den vereinigten Fraktionen wurde in 15 ml-Aliquots auf den Konzentrator gegeben und jeweils für 20 min bei 5000 g und Raumtemperatur in einem swing-out-Rotor zentrifugiert. Das etwa 10-fach aufkonzentrierte Retentat wurde mit PBS 10 x verdünnt und wiederum zentrifugiert. Dieser Waschschritt wurde 2 bis 4 mal wiederholt. Schließlich wurde das Retentat soweit aufkonzentriert, dass eine Konzentration der Antigen-Lösung von 30 bis 60 x bezogen auf das Ausgangsvolumen erreicht wurde. Die Lösung wurde bei -70 °C bis zur weiteren Verwendung aufbewahrt.

### Beispiel 4

### Aufbringen der konzentrierten Borrelia-Antigen-Lösungen auf den Träger Nitrozellulose

Die fertigen *Borrelia*-Antigen-Lösungen wurden über einen Stripe-Prozess auf den Träger Nitrozellulose (Schleicher & Schuell, Dassel, Deutschland) aufgebracht. Hierfür wurden Dispensierapparate der Firma Biodot Ltd. (Huntingdon, GB) nach Angaben des Herstellers verwendet. Die Stripe-Bedingungen wurden so gewählt, dass die Antigen-Lösungen als homogene Linie auf den Träger aufgebracht wurden. Während des Stripe-Prozesses betrug die Luftfeuchtigkeit 40 - 60 % und die Temperatur 19-23 °C. Anschließend wurden die Membranen standardmäßig nach Angaben des Herstellers geblockt.

Die Übertragung der Proteine auf die feste Phase erfolgt elektrophoretisch oder durch direktes Aufbringen auf die immobilisierende Matrix mittels Loch- oder Schlitzmaske (Dot-Blot oder Line-Blot).

### Beispiel 5

### Interpretationskriterien für ein positives Ergebnis

Zur Auswertung der Testergebnisse sind erfindungsgemäß nachfolgende Interpretationskriterien anzuwenden. Die Kriterien entsprechen den Vorgaben des Entwurfes zur neuen Deutschen Industrienorm DIN 58969-44.

Der 1gG-Blot ist dann als positiv zu bewerten, wenn mindestens zwei der folgenden Banden positiv sind: 83 kD-, 58 kD-, 43 kD-, 39 kD-, 30 kD-, 25 kD-, 21 kD-, 17 kD-, 14 kD-Proteinbande, VIsE-Bande. Der IgM-Blot ist dann positiv, wenn mindestens eine der folgenden Banden vorhanden ist: 41 kD-(stark ausgeprägt), 39 kD-, 25 kD-, 17 kD-Proteinbande.

Falls rekombinante Antigene für den IgG-Blot verwendet wurden, ist der IgG-Blot dann positiv, wenn mindestens zwei der folgenden Banden positiv sind: 83 kD-, 58 kD-, 39- kD-, 30 kD, 25 kD-, 21 kD-, 17 kD-Proteinbande, VIsE-, p41 int-Bande. Der IgM-Blot ist dann als positiv zu werten, wenn mindestens zwei der folgenden Banden vorhanden sind: 39 kD-, 25 kD-, 17 kD-Proteinbande, VlsE-, p41 int-Bande. Der IgM-Blot ist ebenfalls positiv, wenn die 25-kD-Bande alleine in starker Ausprägung vorhanden ist.

Fraglich ist das Testergebnis, wenn nur eine Bande im Immunoblot auftritt (außer bei der 25-kD-Proteinbande im IgM-Blot, falls das 25 kD-Protein rekombinant ist).

Wenn keine Banden im Immunoblot angefärbt wurden, ist das Ergebnis als negativ zu bewerten.

### Beispiel 6

### Leistungsmerkmalsdaten des Trägers auf IgG

### Sensitivität:

Aus 68 klinisch definierten Borreliose-positiven Seren wurde die Sensitivität des diagnostischen Trägers bestimmt. Für die frühe Phase der Borreliose-Infektion wurden Patientenseren mit Erythema migrans, Erythema chronicum migrans und Multiple Erythema migrantia untersucht.

Von den Patientenseren im Borreliose-Stadium I mit Erythema migrans (n=29) waren 9 (31%) im 1gG-Blot positiv, 23 (79%) im IgM / IgG-Blot. Im Borreliose-Stadium I mit Erythema chronicum migrans (n=27) waren 12 (44%) im IgG-Blot positiv und 21 (78%) im IgM / IgG-Blot. Von den Seren im Borreliose-Stadium 1 mit Multiple erythema migrantia (n= 13) waren 9 (69%) im IgG-Blot positiv und 10 (91%) im IgM / IgG-Blot positiv.

### Spezifität:

Zur Bestimmung der Spezifität des diagnostischen Trägers wurden 143 Blutspenderseren aus Süddeutschland untersucht. Es wurde eine Spezifität von 140 (98%) ermittelt.

### Beispiel 7

### Leistungsmerkmalsdaten des Trägers auf IgM

### Sensitivität:

Aus 67 klinisch definierten borreliose-positiven Seren wurde die Sensitivität des diagnostischen Trägers bestimmt. Für die frühe Phase der Borreliose-Infektion wurden Patientenseren mit Erythema migrans, Erythema chronicum migrans und Multiple Erythema migrantia untersucht.

Von den Patientenseren im Borreliose-Stadium I mit Erythema migrans (n=29) waren 20 (69%) im IgM-Blot positiv, 23 (79%) im IgM / IgG-Blot. Von den Seren im Borreliose-Stadium I mit Erythema chronicum migrans (n=27) waren 16 (59%) im IgG-Blot positiv und 21 (78%) im IgM / IgG-Blot positiv. Von den Seren im Borreliose-Stadium I mit Multiple erythema migrantia (n= 13) waren 11 (85%) im IgG-Blot positiv und 12 (92%) im IgM / IgG-Blot positiv.

### Spezifität:

Zur Bestimmung der Spezifität des diagnostischen Trägers wurden 140 Blutspenderseren aus Süddeutschland untersucht. Es wurde eine Spezifität von 132 (94%)ermittelt.

### Legende zu den Figuren:

Fig 1: Immunologische Identifizierung von PrepCell-Fraktionen durch analytischen Western-Blot.

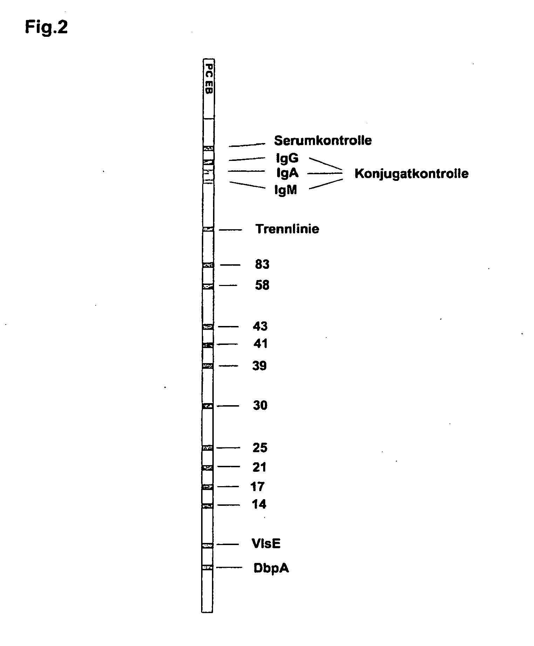

Fig 2: Erfindungsgemäßer Träger mit Anti-*Borrelia*-Antigenen in verschiedenen Konzentration und an verschiedenen Stellen des Trägers aufgebracht, welcher zusätzlich VlsE und DbpA, eine Serumkontrolle, und Konjugat-Kontrollen (IgG, IgM und IgA) umfasst.

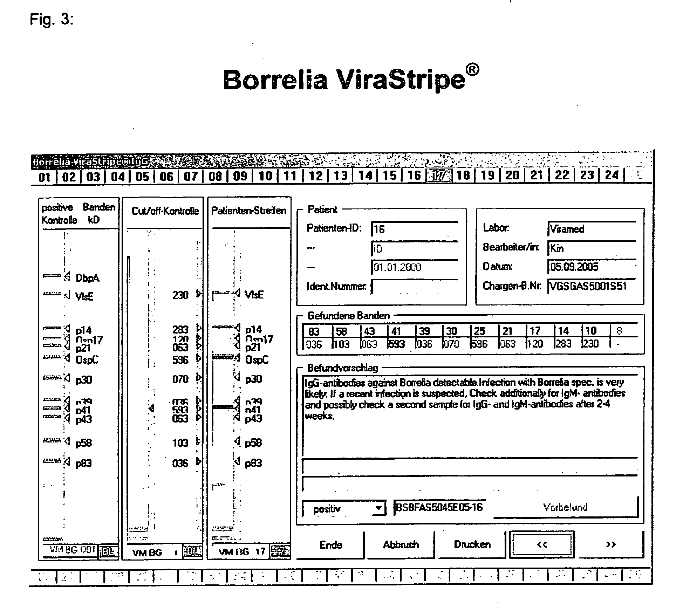

Fig. 3: Verwendung des Trägers in einem automatisierten Lese- und Auswerteverfahren zur Anwendung in der HTS-Diagnostik.

## Patentansprüche

1. Zelllysat-freier Träger zum Nachweis von Anti-*Borrelia*-Antikörpern, umfassend mindestens zehn aus der Membraranfraktion von *Borrelia burgdorferi* sensu lato abgeleitete, und mittels präparativer SDS-Polyacrylamid-Gelelektrophorese aufgereinigte Wildtyp-Antigene, welche an Anti-*Borrelia*-Antikörper binden können und auf unterschiedlichen, voneinander getrennten Positionen des Trägers vorliegen, wobei die aufgereinigten Wildtyp-Antigene in definierter Ortsauflösung oder Positionierung aufgebracht sind und wobei die aufgereinigten Wildtyp-Antigene unabhängig voneinander auf den Träger beladen werden,
wobei Wildtyp-Antigene im Unterschied zu rekombinanten Antigenen posttranslationale Modifikationen aus Borrelia-Zellen aufweisen.

2. Träger nach Anspruch 1, **dadurch gekennzeichnet, dass** dieser mindestens 12 unterschiedliche von *Borrelia burgdorferi* sensu lato abgeleitete aufgereinigte Wildtyp-Antigene umfasst.

3. Träger nach Anspruch 1 oder 2, **dadurch gekennzeichnet, dass** mindestens zwei von *Borrelia burgdorferi* sensu lato abgeleiteten aufgereinigten Wildtyp-Antigene aus der Gruppe bestehend aus 14 kD-, 17 kD-, 21 kD-, 25 kD-, 30 kD-, 39 kD-, 41 kD-, 43 kD-, 58 kD-, 83 kD-Protein und DbpA ausgewählt sind.

4. Träger nach Anspruch 1, 2 oder 3, **dadurch gekennzeichnet, dass** mindestens zwei von *Borrelia burgdorferi* sensu lato abgeleiteten aufgereinigten Wildtyp-Antigene aus der Gruppe bestehend aus 18 kD-, 25 kD- (OspC), 28 kD-, 30 kD-, 31 kD-, 39 kD-, 41 kD-, 45 kD-, 58 KD-, 66 kD-, 83 (93) kD-Protein und DbpA ausgewählt sind.

5. Träger nach einem der Ansprüche 1 bis 4, **dadurch gekennzeichnet, dass** dieser zusätzlich mindestens ein von *Borrelia burgdorferi* sensu lato abgeleitetes rekombinantes Antigen umfasst.

6. Träger nach Anspruch 5, **dadurch gekennzeichnet, dass** das mindestens eine von *Borrelia burgdorferi* sensu lato abgeleitete rekombinante Antigen aus der Gruppe bestehend aus 14 kD-, 17 kD-, 18 kD-, 21 kD-, 25 kD- (OspC), 28 kD- 30 kD-, 31 kD-, 39 kD-, 41 kD-, 43 kD-, 45 kD-, 58 kD-, 66 kD- und 83 kD (93)-Protein, VlsE und DbpA ausgewählt ist.

7. Träger nach Anspruch 5, **dadurch gekennzeichnet, dass** das mindestens eine von *Borrelia burgdorferi* sensu lato abgeleitete rekombinante Antigen VlsE ist.

8. Träger nach einem der Ansprüche 5 bis 7, **dadurch gekennzeichnet, dass** das mindestens eine von *Borrelia burgdorferi* sensu lato abgeleitete rekombinante Antigen ein aufgereinigtes Antigen ist.

9. Träger nach einem der Ansprüche 1 bis 8, **dadurch gekennzeichnet, dass** mindestens 2, oder mindestens 3, oder mindestens 4, oder mindestens 6, oder mindestens 8, oder mindestens 10, oder mindestens 12, oder alle der von *Borrelia burgdorferi* sensu lato abgeleitete aufgereinigte Wildtyp-Antigene in unterschiedlichen Konzentrationen auf dem Träger vorliegen.

10. Träger nach einem der Ansprüche 1 bis 9, **dadurch gekennzeichnet, dass** er zusätzlich mindestens eine Kontrollsubstanz umfasst.

11. Träger nach Anspruch 10, **dadurch gekennzeichnet, dass** die mindestens eine Kontrollsubstanz eine *Borrelia*-unabhängige Konjugatkontrollsubstanz ist, vorzugsweise ausgewählt aus der Gruppe bestehend aus IgA-, IgG- und IgM-Konjugatkontrollsubstanz.

12. Träger nach Anspruch 10 oder 11, **dadurch gekennzeichnet, dass** der Träger mindestens eine Kontaktkontrolle umfasst, die eine Aussage darüber zulässt, ob der Träger mit einer biologischen Probe in Kontakt getreten ist.

13. Träger nach einem der Ansprüche 1 bis 12, **dadurch gekennzeichnet, dass** der Träger ein fester Träger ist, an dem die Antigene immobilisiert sind, wobei der feste Träger vorzugsweise ausgewählt ist aus Platte, Streifen, Membran, Filter, Papier, Film oder Perle.

14. Träger nach Anspruch 13, **dadurch gekennzeichnet, dass** der feste Träger aus einem Material aus der Gruppe umfassend Nitrocellulose, Polyvinylidendifluorid (PVDF), einem Polyamid, Nylon, Celluloseacetat und Polystyren ausgewählt ist.

15. Träger nach einem der Ansprüche 1 bis 14 zur Verwendung in der Diagnostik von Lyme-Borreliose-Infektion oder zur Verlaufskontrolle einer Lyme-Borreliose-Infektion.

16. Verfahren zum Nachweis von Anti-*Borrelia*-Antikörpern umfassend folgende Schritte:
a) Bereitstellen eines Trägers gemäß einem der Ansprüche 1 bis 15;
b) Inkubieren einer biologischen Probe, die möglicherweise Anti-*Borrelia-*Antikörper enthält, mit dem Träger unter Bedingungen, die eine Ausbildung mindestens eines Antikörper-Antigen-Komplexes erlauben; und
c) Bestimmen der Anwesenheit von Anti-*Bornlia*-Antikörpern in der biologischen Probe durch Nachweis mindestens eines Antigen-Antikörper-Komplexes.

17. Verfahren gemäß Anspruch 16, **dadurch gekennzeichnet, dass** mindestens ein Antigen spezifisch mit einem Anti-*Borrelia*-Antikörper reagiert.

18. Verfahren gemäß einem der Ansprüche 16 oder 17, **dadurch gekennzeichnet, dass** die biologische Probe von einem Tier oder einem Menschen abgeleitet ist.

19. Verfahren gemäß Anspruch 18, **dadurch gekennzeichnet, dass** die aus einem Tier oder Menschen abgeleitete biologische Probe eine flüssige Probe ist, vorzugsweise Blut, Plasma, Serum, Urin, Liquor, Speichel, verflüssigte Gewebeprobe oder Synovialflüssigkeit.

20. Testkit zur Verwendung in der Diagnostik von Lyme-Borreliose-Infektion oder zur Verlaufskontrolle einer Lyme-Borreliose-Infektion umfassend den Träger gemäß einem der Ansprüche 1 bis 15 und zusätzlich mindestens eine Substanz zum Nachweis eines Antigen-Antikörper-Komplexes.

21. Testkit nach Anspruch 20, **dadurch gekennzeichnet, dass** der Testkit auch eine Anweisung zur Auswertung von gewonnenen Testergebnissen enthält.

## Claims

1. Cell-lysate-free carrier for the detection of anti-*Borrelia* antibodies, comprising at least ten wild-type antigens which are derived from the membrane fraction of *Borrelia burgdorferi* sensu lato and cleaned by preparative SDS-polyacrylamide gel electrophoresis and which can bind to anti-*Borrelia* antibodies and are present on different carrier positions separated from each other, wherein the cleaned wild-type antigens are applied in a defined spatial resolution or positioning and wherein the cleaned wild-type antigens are loaded independently of each other on the carrier, wherein wild-type antigens in contrast to recombinant antigens comprise post-translational modifications of Borrelia cells.

2. Carrier according to Claim 1, **characterised in that** said carrier comprises at least 12 different cleaned wild-type antigens derived from *Borrelia burgdorferi* sensu lato.

3. Carrier according to Claim 1 or 2, **characterised in that** the at least two cleaned wild-type antigens derived from *Borrelia burgdorferi* sensu lato are selected from the group consisting of 14 kD, 17 kD, 21 kD, 25 kD, 30 kD, 39 kD, 41 kD, 43 kD, 58 kD, 83 kD protein and DbpA.

4. Carrier according to Claim 1, 2 or 3 **characterised in that** the at least two cleaned wild-type antigens derived from *Borrelia burgdorferi* sensu lato are selected from the group consisting of 18 kD, 25 kD (OspC), 28 kD, 30 kD, 31 kD, 39 kD, 41 kD, 45 kD, 58 kD, 66 kD, 83 (93) kD protein and DbpA.

5. Carrier according to one of Claims 1 to 4, **characterised in that** said carrier comprises additionally at least one recombinant antigen derived from *Borrelia burgdorferi* sensu lato.

6. Carrier according to Claim 5, **characterised in that** the at least one recombinant antigen derived from *Borrelia burgrdorfieri* sensu lato is selected from the group consisting of 14 kD, 17 kD, 18 kD, 21 kD, 25 kD (OspC), 28 kD, 30 kD, 31 kD, 39 kD, 41 kD, 43 kD, 45 kD, 58 kD, 66 kD and 83 kD (93) protein, VIsE and DbpA.

7. Carrier according to Claim 5, **characterised in that** the at least one recombinant antigen derived from *Borrelia burgdorferi* sensu lato is VIsE.

8. Carrier according to one of Claims 5 to 7, **characterised in that** the at least one recombinant antigen derived from *Borrelia burgdorferi* sensu lato is a cleaned antigen.

9. Carrier according to one of Claims 1 to 8, **characterised in that** at least 2, or at least 3, or at least 4, or at least 6, or at least 8, or at least 10, or at least 12, or all of the cleaned wild-type antigens derived from *Borrelia burgdorferi* sensu lato are present on the carrier in different concentrations.

10. Carrier according to one of the Claims 1 to 9, **characterised in that** it comprises additionally at least one control substance.

11. Carrier according to Claim 10, **characterised in that** the at least one control substance is a *Borrelia*-independent conjugate control substance, preferably selected from the group consisting of IgA, IgG and IgM conjugate control substances.

12. Carrier according to Claim 10 or 11, **characterised in that** the carrier comprises at least one contact control which allows a statement to be made as to whether the carrier has been in contact with a biological sample.

13. Carrier according to one of the Claims 1 to 12, **characterised in that** the carrier is a fixed carrier on which the antigens are immobilised, wherein the fixed carrier is preferably selected from plate, strip, membrane, filter, paper, film or pearl.

14. Carrier according to Claim 13, **characterised in that** the fixed carrier is selected from a material from the group comprising nitrocellulose, polyvinylidene difluoride (PVDF), a polyamide, nylon, cellulose acetate and polystyrene.

15. Carrier according to one of the Claims 1 to 14 for use in the diagnosis of Lyme borreliosis infection or for monitoring the progress of a Lyme borreliosis infection.

16. Method for the detection of anti-*Borrelia* antibodies comprising the following steps:
a) provision of a carrier according to one of the Claims 1 to 15;
b) incubation of a biological sample which may contain anti-*Borrelia* antibodies with the carrier under conditions which allow the formation of at least one antibody-antigen complex; and
c) determination of the presence of anti-*Borrelia* antibodies in the biological sample through the detection of at least one antigen-antibody complex.

17. Method according to Claim 16, **characterised in that** at least one antigen reacts specifically with an anti-*Borrelia* antibody.

18. Method according to one of the Claims 16 or 17, **characterised in that** the biological sample has been derived from an animal or a human.

19. Method according to Claim 18, **characterised in that** the biological sample derived from an animal or human is a liquid sample, preferably blood, plasma, serum, urine, liquor, saliva, liquefied tissue sample or synovial fluid.

20. Test kit for use in the diagnosis of Lyme borreliosis infection or for monitoring the progress of a Lyme borreliosis infection comprising the carrier according to one of the Claims 1 to 15 and additionally at least one substance for the detection of an antigen-antibody complex.

21. Test kit according to Claim 20; **characterised in that** the test kit also contains instructions for the evaluation of the test results obtained.

## Revendications

1. Support sans lysat cellulaire pour la détection d'anticorps anti-*Borrelia*, comprenant au moins dix antigènes de type sauvage issus de la fraction membranaire de *Borrelia burgdorferi* sensu lato et purifiés par électrophorèse préparative sur gel de polyacrylamide en présence de SDS, lesquels antigènes peuvent se lier à des anticorps anti-*Borrelia* et être disponibles en différentes positions du support, distinctes les unes des autres, de sorte que les antigènes de type sauvage purifiés sont présents avec une résolution locale ou un positionnement défini et de sorte que les antigènes de type sauvage purifiés sont chargés sur le support indépendamment les uns des autres, les antigènes de type sauvage présentant, à la différence des antigènes recombinants, des modifications post-traductionnelles des cellules Borrelia.

2. Support selon la revendication 1, **caractérisé en ce qu'**il comporte au moins 12 différents antigènes de type sauvage purifiés, issus de *Borrelia burgdorferi* sensu lato.

3. Support selon la revendication 1 ou 2,
**caractérisé en ce qu'**au moins deux antigènes de type sauvage purifiés, issus de *Borrelia burgdorferi* sensu lato sont choisis dans le groupe constitué des protéines de 14 kD, 17 kD, 21 kD, 25 kD, 30 kD, 39 kD, 41 kD, 43 kD, 58 kD, 83 kD et de DbpA.

4. Support selon la revendication 1, 2 ou 3, **caractérisé en ce qu'**au moins deux antigènes de type sauvage purifiés, issus de *Borrelia burgdorferi* sensu lato sont choisis dans le groupe constitué des protéines de 18 kD, 25 kD (OspC), 28 kD, 30 kD, 31 kD, 39 kD, 41 kD, 45 kD, 58 kD, 66 kD, 83 (93) kD et de DbpA.

5. Support selon l'une des revendications 1 à 4, **caractérisé en ce qu'**il comprend en outre au moins un antigène recombinant issu de *Borrelia burgdorferi* sensu lato.

6. Support selon la revendication 5, **caractérisé en ce que** le au moins un antigène recombinant issu de *Borrelia burgdorferi* sensu lato est choisi dans le groupe constitué des protéines de 14 kD, 17 kD, 18 kD, 21 kD, 25 kD (OspC), 28 kD, 30 kD, 31 kD, 39 kD, 41 kD, 43 kD, 45 kD, 58 kD, 66 kD et 83 (93) kD, de VlsE et de DbpA.

7. Support selon la revendication 5, **caractérisé en ce que** le au moins un antigène recombinant issu de *Borrelia burgdorferi* sensu lato est VlsE.

8. Support selon l'une des revendications 5 à 7, **caractérisé en ce que** le au moins un antigène recombinant issu de *Borrelia burgdorferi* sensu lato est un antigène purifié.

9. Support selon l'une des revendications 1 à 8, **caractérisé en ce qu'**au moins 2, ou au moins 3, ou au moins 4, ou au moins 6, ou au moins 8, ou au moins 10, ou au moins 12, ou la totalité des antigènes de type sauvage purifiés, issus de *Borrelia burgdorferi* sensu lato sont présents sur le support à différentes concentrations.

10. Support selon l'une des revendications 1 à 9, **caractérisé en ce qu'**il comprend en outre au moins une substance de contrôle.

11. Support selon la revendication 10, **caractérisé en ce que** la au moins une substance de contrôle est une substance de contrôle conjuguée indépendante de *Borrelia,* de préférence choisie dans le groupe constitué de substances de contrôle conjuguées avec IgA, IgG et IgM.

12. Support selon la revendication 10 ou 11, **caractérisé en ce qu'**il comprend au moins un témoin de contact, qui permet en outre de déterminer si le support est entré en contact avec un échantillon biologique.

13. Support selon l'une des revendications 1 à 12, **caractérisé en ce qu'**il s'agit d'un support solide sur lequel sont immobilisés les antigènes, de sorte que le support solide est de préférence choisi entre une plaque, une bande, une membrane, un filtre, un papier, un film ou des billes.

14. Support selon la revendication 13, **caractérisé en ce que** le support solide est en un matériau choisi dans le groupe constitué de la nitrocellulose, du poly(fluorure de vinylidène) (PVDF), d'un polyamide, du nylon, d'acétate de cellulose et de polystyrène.

15. Support selon l'une des revendications 1 à 14 pour une utilisation dans le diagnostic d'une infection à la borreliose de Lyme ou pour le contrôle de l'évolution d'une infection à la borreliose de Lyme.

16. Procédé pour la détection d'anticorps anti-*Borrelia* comprenant les étapes suivantes consistant à :
a) préparer un support selon l'une des revendications 1 à 15 ;
b) incuber un échantillon biologique contenant éventuellement des anticorps anti-*Borrelia*, avec le support dans des conditions qui permettent la formation d'au moins un complexe antigène-anticorps ; et
c) déterminer la présence d'anticorps anti-*Borrelia* dans l'échantillon biologique par la détection d'au moins un complexe antigène-anticorps.

17. Procédé selon la revendication 16, **caractérisé en ce qu'**au moins un antigène réagit spécifiquement à un anticorps anti-*Borrelia*.

18. Procédé selon l'une des revendications 16 et 17, **caractérisé en ce que** l'échantillon biologique est issu d'un animal ou d'un être humain.

19. Procédé selon la revendication 18, **caractérisé en ce que** l'échantillon biologique issu d'un animal ou d'un être humain est un échantillon liquide, de préférence du sang, du plasma, du sérum, de l'urine, du liquide céphalo-rachidien, de la salive, des échantillons tissulaires liquéfiés ou du liquide synovial.

20. Kit de test pour une utilisation dans le diagnostic d'une infection à la borreliose de Lyme ou pour le contrôle de l'évolution d'une infection à la borreliose de Lyme, comprenant le support selon l'une des revendications 1 à 15 et en outre au moins une substance pour détecter un complexe antigène-anticorps.

21. Kit de test selon la revendication 20, **caractérisé en ce qu'**il contient également des instructions pour exploiter les résultats de test obtenus.
